# EUROPÄISCHE PATENTSCHRIFT

(11) **EP 4 286 852 B1**
(45) Veröffentlichungstag und Bekanntmachung des Hinweises auf die Patenterteilung: **14.08.2024**
(21) Anmeldenummer: 22212909.0
(22) Anmeldetag: 12.12.2022
(51) Int. Cl.: G01N 33/569, G01N 33/58, C12Q 1/04, C12Q 1/689

(54) **VERFAHREN ZUM ERKENNEN EINES BIOFILMS IN GESCHLOSSENEN ANLAGEN**
METHOD FOR DETECTING BIOFILM IN CLOSED SYSTEMS
PROCÉDÉ DE DÉTECTION D'UN BIOFILM DANS DES INSTALLATIONS FERMÉES

(30) Priorität: 03.06.2022 AT 503942022
(43) Veröffentlichungstag der Anmeldung: 06.12.2023
(73) Patentinhaber: WG Holding GmbH, 4863 Seewalchen am Attersee (AT)
(72) Erfinder: Gauges, Wolfgang, 4863 Seewalchen (AT)
(74) Vertreter: Hübscher & Partner Patentanwälte GmbH

(56) Entgegenhaltungen:
- DE-A1- 102017 005 695
- US-A1- 2010 112 630

## Beschreibung

Die Erfindung bezieht sich auf ein Verfahren zum Erkennen eines Biofilms in geschlossenen Anlagen, bei dem zuerst ein Nachspülmedium in die entleerte Anlage eingebracht und anschließend eine enzymatische Lösung zum Anlösen des Biofilms beigemischt wird und das Gemisch in der Anlage zirkuliert. Biofilme umfassen eine Schleimschicht, werden von Mikroorganismen in wässrigen Umgebungen gebildet und besiedeln und kontaminieren geschlossene Anlagen, wie sie insbesondere in der Lebensmittelproduktion verwendet werden. Da Biofilme wasserabweisende Grenzschichten umfassen und deswegen nicht einfach aus der Anlage gespült werden können, ist der Reinigungsaufwand für eine Anlage dementsprechend höher. Aus diesem Grund sollte vor einer Reinigung ermittelt werden, ob tatsächlich eine Biofilmkontamination vorliegt, um unnötige Produktionsausfälle und aufwändige Reinigungsprozesse zu vermeiden.

Aus dem Stand der Technik sind Verfahren zur Erkennung von Biofilmen bekannt. Die US-amerikanische Patentanmeldung US2010/112630 A1 enthüllt ein Verfahren zum Erkennen eines Biofilms. DE102017005695A1 zeigt ein solches Verfahren, bei dem eine Produktionsanlage zur Lebensmittelherstellung einen Messkreislauf aufweist, von dem ein kleinerer Spülkreislauf abgezweigt wird. In diesem Spülkreislauf wird Spülwasser als Nachspülmedium verwendet, welchem ein Enzymreiniger zum Anlösen eines eventuell vorhandenen Biofilms beigemischt wird. Danach wird ein erster TOC (total organic carbon)-Wert für dieses Gemisch bestimmt und das Gemisch wieder dem größeren Messkreislauf zugeführt. Nun wird der TOC-Wert des größeren Messkreislaufs als zweiter TOC-Wert bestimmt und anschließend mit dem ersten TOC-Wert verglichen. Ist der zweite TOC-Wert höher als der erste, wird ein Biofilm detektiert. Sind beide TOC-Werte gleich hoch, wird kein Biofilm detektiert.

Nachteilig am Stand der Technik ist aber, dass zur Erkennung des Biofilms davon ausgegangen wird, dass die beim TOC-Wert gemessene Konzentration des gesamten organisch gebundenen Kohlenstoffs im Spülwasser von einem Biofilm stammt. Der gemessene Kohlenstoff muss jedoch nicht zwangsweise als Biofilm vorliegen, da auch andere organische Verunreinigungen den TOC-Wert beeinflussen. Darüber hinaus müssen die Volumina des Mess- und Spülkreislaufs sowie die Menge an Enzymreinigerkonzentration bestimmt und zwei TOC-Werte gemessen und abgeglichen werden.

Der Erfindung liegt somit die Aufgabe zugrunde, trotz anderer organischer Verunreinigungen und mit einfacher Aufbereitung des zu messenden Nachspülmediums zuverlässig das Vorhandensein eines Biofilms zu detektieren.

Die Erfindung löst die gestellte Aufgabe dadurch, dass der Anlage eine Probe des zirkulierten Gemischs entnommen wird, welche mit Fluoreszenzmarkern fluoreszenzmarkiert wird, die eine Biofilmkomponente spezifisch binden und ein vorzugsweise photometrisches Abbild der Probe beispielsweise mittels Fluoreszenzmikroskopie oder einem Fotometer erstellt wird, wonach ein Erkennungssignal dann ausgegeben wird, wenn im Abbild die charakteristischen Emissionswellenlängen jener Fluoreszenzmarker detektiert werden, mit der die in der Probe vorhandenen fluoreszenzmarkierten Biofilmkomponenten markiert sind. Der Erfindung liegt dabei die Überlegung zugrunde, dass zum Erkennen eines Biofilmes nicht die Konzentration des organischen Kohlenstoffes im zirkulierten Gemisch bestimmt wird, sondern die für den Biofilm charakteristischen Biofilmkomponenten, also beispielsweise Zellen, Teile der DNS solcher Zellen und extrazelluläre dreidimensionale Strukturen, wie beispielsweise extrazelluläre polymere Substanzen (EPS), die von den Zellen gebildet werden. Durch die Verwendung üblicher Protokolle der Fluoreszenzmarkierung können die verschiedenen Komponenten des Biofilms spezifisch mit Fluoreszenzmarkern markiert und durch anschließende Techniken, wie beispielsweise Fluoreszenzmikroskopie oder Fotometrie detektierbar und unterscheidbar gemacht werden. Überschüssige Fluoreszenzmarker, oder solche, für die in der Probe kein Bindungspartner vorhanden ist, werden anschließend unter Berücksichtigung der üblichen Protokolle ausgewaschen. So kann beispielsweise die für eine lebende Zelle typische Esterase-Aktivität mit einem Fluoreszenzmarker, das für tote Zellen typische Versagen der Plasmamembranintegrität mit einem weiteren Fluoreszenzmarker und die EPS mit einem weiteren Fluoreszenzmarker versehen werden. Werden verschiedene Fluoreszenzmarker verwendet, können diese anhand ihrer voneinander unterscheidbaren, charakteristischen Emissionswellenlängen einfach und vorzugsweise automatisiert detektiert werden, um so die unterschiedlichen Biofilmkomponenten, beispielsweise lebende Zellen, tote Zellen und extrazellulären dreidimensionalen Strukturen in der zirkulierten Probe zu unterscheiden. Aus der gemessenen Fluoreszenz kann ermittelt werden, ob in der Probe ein Biofilm vorliegt und anschließend ein Erkennungssignal ausgegeben werden, nämlich dann, wenn die charakteristischen Emissionswellenlängen der Fluoreszenzmarker zum Markieren der charakteristischen Biofilmkomponenten detektiert werden. Weiters kann beispielsweise bestimmt werden, ob die Zellen, die einen Teil eines eventuell vorhandenen Biofilms bilden, noch leben oder bereits tot sind, nämlich, wenn die Fluoreszenzmarker zum Markieren der lebenden bzw. toten Zellen detektiert werden. Über die gemessene Fluoreszenz der verschiedenen Fluoreszenzmarker kann bereits einfach und vorzugsweise automatisiert und ohne Kalibration das quantitative Verhältnis, also die gemessene Quanten- oder Lichtausbeute für jeden Fluoreszenzmarker, zwischen den verschiedenen Biofilmkomponenten bestimmt werden. Vorzugsweise kann der Benutzer zusätzlich selbst aufgrund der unterschiedlichen Fluoreszenzmarkierungen eventuell vorhandene dreidimensionale Strukturen und/oder den charakteristischen Zellverbund oder andere Biofilmkomponenten im Fluoreszenzbild erkennen und dadurch qualitativ feststellen, ob ein Biofilm vorhanden ist. Dazu kann er beispielsweise das Fluoreszenzbild mit Referenzbildern abgleichen. Vorzugsweise wird auch diese qualitative Bestimmung automatisiert durch für die Bildklassifikation übliche neuronale Netze, durchgeführt. Solche Neuronalen Netze können vorab mit Hilfe von von einem Benutzer klassifizierten Abbildern trainiert werden. Beispielsweise kann das Erkennungssignal, insbesondere bei der automatisierten Bestimmung, dann ausgegeben, wenn die Lichtausbeute eines zu detektierenden Fluoreszenzmarkers einen vorgegebenen Schwellenwert überschreitet und somit eine Mindestmenge an einer Biofilmkomponente nachgewiesen wird. Ein weiterer Vorteil des Verfahrens liegt darin, dass die Entnahme der zirkulierten Probe im Rahmen eines Cleaning in Place (CIP)-Verfahrens zur Reinigung der Anlage durchgeführt werden kann und die Anlage nach Probenentnahme weiter gereinigt werden kann. Das Nachspülmedium kann Nachspülwasser sein, welches auch im Rahmen eines CIP-Verfahrens eingesetzt wird. Da die enzymatische Lösung in das gesamte in der Anlage vorhandene Nachspülmedium eingebracht wird, muss die korrekte Menge und Konzentration an enzymatischer Lösung nur einmal für ein relativ großes Volumen erfolgen, wodurch Messungenauigkeiten bei der Beimischung und Konzentration relativ einfach korrigiert werden können. Als Fluoreszenzmarker wird jedes Biomolekül verstanden, welches spezifisch an die gewünschte Biofilmkomponente binden kann und entweder direkt oder indirekt Lichtemissionen in einem gewissen Wellenlängenbereich erzeugt. Direkte Emission bedeutet in diesem Zusammenhang, dass der Fluoreszenzmarker nach Anregung in einem gewissen Wellenlängenbereich selbst Licht emittiert. Indirekte Emission bedeutet, dass der Fluoreszenzmarker mit einer weiteren Substanz interagiert, sodass diese weitere Substanz aufgrund dieser Interaktion Licht in einem gewissen Wellenlängenbereich emittiert.

Um den Biofilm nicht nur quantitativ über die Lichtausbeute, sondern auch qualitativ detektierbar zu machen und dabei vorzugsweise die Menge an benötigten Fluoreszenzmarkern zu reduzieren, wird vorgeschlagen, dass das Abbild der Probe mittels Fluoreszenzmikroskopie erstellt wird. Die enzymatische Lösung ist anhand bekannter stöchiometrischer Verfahren so gemischt und dosiert, dass sie den eventuell vorhandenen Biofilm nur anlöst und nicht zersetzt. Das bedeutet, dass wenigstens ein Teil der dreidimensionalen Struktur des Biofilms erhalten bleibt und in der Probe über die Detektion mittels Fluoreszenzmikroskopie nachweisbar ist. Dadurch kann der Biofilm über seine dreidimensionale Struktur von einem Benutzer selbst dann nachgewiesen werden, wenn nur ein verhältnismäßig geringer Anteil der markierten Biofilmkomponente an einen Fluoreszenzmarker gebunden wurde. Durch die bei der Fluoreszenzmikroskopie erhaltene zusätzliche Information der räumlichen Verteilung des Fluoreszenzmarkers ist es nämlich nicht mehr notwendig, die Biofilmkomponente unter Verwendung üblicher Protokolle mit Fluoreszenzmarker zu übersättigen, um dessen Vorhandensein festzustellen. Auch hier kann ein neuronales Netz auf der Basis von Referenzbildern trainiert werden, die dreidimensionalen Strukturen eines Biofilms zu erkennen und bei Erkennen das Erkennungssignal auszugeben.

Um das enzymatische Anlösen mit einfachen technischen Mitteln zu beschleunigen, kann das Nachspülmedium vor dem Beimischen der enzymatischen Lösung auf 40-50°C temperiert werden. Über die Temperatur des Nachspülmediums thermische Energie bereitgestellt, die die Reaktion zwischen Biofilm und Enzym begünstigt und dementsprechend das enzymatische Anlösen des Biofilms erleichtert. Gleichzeitig wird bei den vorgeschlagenen Temperaturen ein Denaturieren der Enzyme vermieden.

Um vorab zu bestimmen, ob die Probe fluoreszenzmarkiert werden muss, um einen Biofilm zu detektieren, kann der Anteil organischer Rückstände in der Probe vor dem Erstellen des Abbildes quantitativ bestimmt werden und kein Biofilm detektiert werden, wenn der Anteil einen vorgegebenen Grenzwert unterschreitet. Dem liegt die Überlegung zugrunde, dass das Überschreiten eines vorgegebenen Grenzwerts an organischen Rückständen eine Voraussetzung für das eventuelle Vorhandensein eines Biofilms ist, da sich dieser nur dann ausbilden kann, wenn ausreichend organische Rückstände in der Probe vorhanden sind. Wird demzufolge keine ausreichende Konzentration an organischen Rückständen gemessen, kann bereits bestimmt werden, dass sich kein Biofilm ausgebildet haben kann und das Verfahren kann vor Durchführung der Fluoreszenzmarkierung beendet werden. Der Grenzwert kann beispielsweise durch mehrere Messungen des Anteils an organischen Rückständen an einer Probe mit vorhandenem Biofilmen erstellt werden. Die Messung kann beispielsweise eine TOC-Messung oder eine chemische Sauerstoffbedarfs (CSB-) Messung sein.

Die enzymatische Lösung kann Amylasen und/oder Proteasen und/oder Saccharasen umfassen, da diese Enzyme die Aktivierungsenergie zur Aufspaltung der Hauptbestandteile des Biofilms herabsetzen.

Um ein ausreichendes Anlösen eines eventuell vorhandenen Biofilms durch die enzymatische Lösung so zu fördern, dass dieser zuverlässig detektiert werden kann, kann das Gemisch vor der Probenentnahme bei konstanter Temperatur wenigstens eine Stunde in der geschlossenen Anlage zirkulieren. Dadurch wird bei konstanten Reaktionsbedingungen genügend Zeit zur Verfügung gestellt, dass die enzymatische Reaktion selbst bei verhältnismäßig geringen Mengen an Enzym im Nachspülmedium so ablaufen kann, dass eventuell vorhandener Biofilm in der Probe nachweisbar ist.

Die Reaktionsgeschwindigkeit eines Enzyms hängt auch vom pH-Wert der Reaktionsumgebung ab. So kann sich die maximale Reaktionsgeschwindigkeit für verschiedene Enzyme bei unterschiedlichen pH-Werten einstellen. Um die enzymatischen Reaktionen auch dann zu begünstigen, wenn verschiedene Enzyme in der enzymatischen Lösung vorhanden sind, wird daher vorgeschlagen, dass der pH-Wert des Gemisches verändert wird und dass Gemisch mit verändertem pH-Wert wenigstens eine Stunde in der geschlossenen Anlage zirkuliert, bevor die Probe entnommen wird. Zufolge dieser Maßnahmen kann der pH-Wert des Gemischs so eingestellt werden, dass die Reaktionsgeschwindigkeit eines oder mehrerer der im enzymatischen Gemisch vorhandenen Enzyme optimiert wird. In einer bevorzugten Ausführungsform des Verfahrens wird das Gemisch zuerst bei einem ersten pH-Wert für eine erste Zeitspanne zirkuliert und anschließend bei einem zweiten pH-Wert für eine zweite Zeitspanne zirkuliert, um zuerst die Reaktionsgeschwindigkeit eines ersten Enzyms und anschließend die Reaktionsgeschwindigkeit eines zweiten Enzyms zu maximieren. So kann das Gemisch beispielsweise zuerst mit unbeeinflusstem pH-Wert zirkulieren und anschließend der pH-Wert angehoben werden, wonach das Gemisch weiter zirkuliert. Der pH-Wert kann beispielsweise auf 10 angehoben werden.

Um beim Erstellen des Abbilds der Probe eine höhere Quantenausbeute zu erreichen und dadurch das Signal-zu-Rausch-Verhältnis zu erhöhen, wird vorgeschlagen, dass die Probe ein durch Filtration zumindest eines Teils des zirkulierten Gemischs gewonnener Filterkuchen ist. Durch die Filtration wird die Konzentration der Biofilmkomponenten in einem gegebenen Probenvolumen erhöht, wodurch mehr Bindungspartner für die Fluoreszenzmarkierung vorhanden sind. Durch die daraus resultierende erhöhte Quantenausbeute kann mehr von den spezifischen Fluoreszenzmarkern emittiertes Licht detektiert werden, was sowohl die quantitative als auch die qualitative Analyse der Probe erleichtert.

Um nicht nur das Vorhandensein eines Biofilms zu detektieren, sondern in weiterer Folge auch den Biofilm bildende Pathogene zu bestimmen, wird vorgeschlagen, dass ein Teil der Probe einem Polymerasekettenreaktionsprozess unterzogen wird, bei dem ein bestimmter Desoxyrobonukleinsäurestrangabschnitt als Biofilmkomponente vervielfältigt wird. Zufolge dieser Maßnahmen kann den üblichen Polymerasekettenreaktionsprozess (PCR) - Protokollen folgend ein Abschnitt der Desoxyribonukleinsäure (DNS) der Pathogene ausgewählt und durch PCR vervielfältigt werden. Durch die Vervielfältigung des Abschnitts kann dieser bei Vorhandensein in der Probe einfacher detektiert werden, wodurch die diesen Abschnitt aufweisenden Pathogene in der Probe nachgewiesen werden können. Zwar lassen sich durch die Verwendung eines PCR-Verfahrens keine genauen Aussagen über die ursprüngliche Quantität des Abschnitts der DNS in der unbehandelten Probe mehr treffen, allerdings kann das Vorhandensein des Abschnitts der DNS zufolge dieser Maßnahmen bereits bei geringen Mengen an Biofilmkomponenten und somit in einem frühen Stadium nachgewiesen werden. Dabei spielt es grundsätzlich keine Rolle, ob das PCR-Verfahren vor, während oder nach dem Erstellen des Abbilds der Probe durchgeführt wird. So kann die Bestimmung des Biofilms und der Pathogene besonders schnell durchgeführt werden, wenn das Abbilds zeitlich parallel zum PCR-Verfahren erstellt wird. Andererseits kann die Bestimmung ressourcenschonender erfolgen, wenn das PCR-Verfahren erst dann durchgeführt wird, wenn durch das Erstellen des Abbilds und das darauffolgende Erkennungssignal das Vorhandensein eines Biofilms detektiert wurde.

Um sowohl eine spezifische als auch genaue Markierung der spezifischen Biofilmkomponenten zu erlauben und das Abbild der Probe mit technisch einfachen Mittel auszuwerten, wird vorgeschlagen, dass das Abbild der Probe als Teil eines enzymgebundenen Immunosorbentnachweisverfahrens erstellt wird. Bei einem solchen enzymgebundenen Immunosorbentnachweisverfahren (ELISA) wird zuerst ein unmarkierter Antikörper an einem Substrat, beispielsweise einer funktionalisierten Oberfläche, gebunden und dadurch immobilisiert. Dieser unmarkierte Antikörper, weist eine spezifische Bindungsstelle für die Biofilmkomponente auf. Üblicherweise variiert die Oberflächenkonzentration dieses Antikörpers abschnittsweise auf dem Substrat, um eine quantitative Bestimmung der Biofilmkomponente zu vereinfachen, sodass die nun folgenden Schritte für mehrere Abschnitte durchgeführt werden können. Anschließend wird den gebundenen Antikörpern zumindest ein Teil der Probe zugeführt, sodass die in der Probe vorhandenen Biofilmkomponenten spezifisch an dem immobiliserten Antikörper binden. Nach dem folgenden Waschschritt wird ein Detektions-Antikörper, der ein Enzym umfasst, hinzugegeben, der ebenso spezifisch, aber an einem anderen Epitop an der Biofilmkomponente bindet. Überschüssige Detektions-Antikörper werden wiederum in einem weiteren Waschschritt entfernt. Anschließend wird ein Detektionssubstrat beigegeben, welches bei Durchlaufen einer enzymatischen Reaktion mit dem Enzym des Detektions-Antikörpers Licht emittiert. Somit emittiert der Detektions-Antikörper nach dem oben beschriebenen Prinzip indirekt Licht. Die Quantität des enzymatisch abreagierten und somit Licht emittierenden Detektionssubstrats ist unter anderem proportional zur Quantität der gebundenen Biofilmkomponente und kann somit zum Nachweis dieser dienen. Die Lichtausbeute kann in diesem Fall mittels Fluoreszenzmikroskopie bestimmt werden. In einer bevorzugten Ausführungsform wird allerdings ein Photometer verwendet, da mit diesem die Signalstärke genauer bestimmbar ist.

Selbst bei der Verwendung spezifischer Antikörper können bei komplexen Proben wie Biofilmen, die aus einer Vielzahl an verschiedenen Biomolekülen zusammengesetzt sind, unerwünschte Bindungen und daher eine Verfälschung des Messergebnisses auftreten. Um ohne weitere Vorbehandlungsschritte trotzdem eine hohe Spezifizität und damit eine hohe Messgenauigkeit zu erreichen, wird daher vorgeschlagen, dass das enzymgebundene Immunosorbentnachweisverfahrens ein Sandwich- enzymgebundenes Immunosorbentnachweisverfahren ist. Beim Sandwich- enzymgebundenen Immunosorbentnachweisverfahren bindet der Detektions-Antikörper nicht direkt an der Biofilmkomponente, sondern an einen an der Biofilmkomponente gebundenen Zwischen-Antikörper. Die Bindung des Zwischen-Antikörpers an der Biofilmkomponente und die Bindung des Detektions-Antikörpers am Zwischen-Antikörper, resultiert in einer erhöhten Gesamtspezifizität und einer räumlichen Trennung des Detektions-Antikörpers von der Biofilmkomponente. Dadurch wird die Messgenauigkeit erhöht, da Fehlbindungen des Detektions-Antikörpers reduziert werden können.

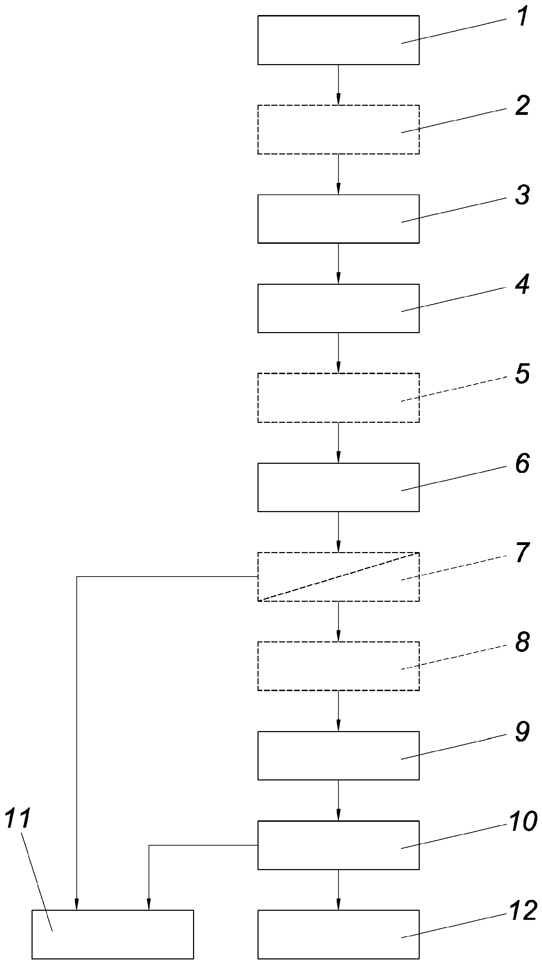

In der Zeichnung ist der Erfindungsgegenstand beispielsweise dargestellt. Die Zeichnung zeigt in der
- Fig. 1: eine schematische Darstellung der Verfahrensschritte eines erfindungsgemäßen Verfahrens, wobei optionale Verfahrensschritte strichliert dargestellt sind.

Ein erfindungsgemäßes Verfahren zum Erkennen eines Biofilms in geschlossenen Anlagen umfasst in einem ersten Verfahrensschritt 1, in dem die Anlage geleert und anschließend mit einem Nachspülmedium befüllt wird. Das Nachspülmedium kann bevorzugter Weise Nachspülwasser sein. Dies ermöglicht beispielsweise ein einfaches Einstellen des pH-Werts des Nachspülmediums und/oder eine an das Verfahren anschließende CIP-Reinigung der Anlage. Anschließend kann in einem optionalen Verfahrensschritt 2 das Nachspülmedium auf eine Temperatur zwischen 40°C und 50°C temperiert werden, damit das Nachspülmedium bei der folgenden Beimischung einer enzymatischen Lösung in Verfahrensschritt 3 bereits in thermischem Gleichgewicht ist und die Reaktionsgeschwindigkeit der Enzyme in der enzymatischen Lösung aufgrund der verfügbaren thermischen Energie beschleunigt wird. Die Beimischung der enzymatischen Lösung in Verfahrensschritt 3 kann auch sequenziell erfolgen. Es können also mehrere enzymatische Lösungen hintereinander beigemischt werden. Um den Enzymen ausreichend Zeit zu geben, um einen eventuell vorhandenen Biofilm anzulösen, wird das durch die Zugabe der enzymatischen Lösung zum Nachspülwasser entstehende Gemisch in Verfahrensschritt 4 in der geschlossenen Anlage zirkuliert. In einer bevorzugten Ausführungsform des Verfahrens wird das Gemisch bei konstanter Temperatur für wenigstens eine Stunde zirkuliert.

Da verschiedene Enzyme bei verschiedenen pH-Werten ihre höchste Reaktionsgeschwindigkeit erreichen, kann der pH-Wert des Gemischs in einem optionalen Verfahrensschritt 5 geändert werden, um die Reaktionsgeschwindigkeit spezifischer Enzyme zu erhöhen. Auch in diesem Fall wird das Gemisch bei verändertem pH-Wert für wenigstens eine Stunde zirkuliert, um den Enzymen ausreichend Zeit zum Anlösen eines etwaigen Biofilms zu geben. In einer bevorzugten Ausführungsform kann der pH-Wert des Gemischs mehrfach verändert werden um die optimalen Reaktionsgeschwindigkeiten verschiedener Enzyme nacheinander zu erreichen. Die enzymatische Lösung ist so zusammengesetzt, dass durch die Enzymaktivität der Biofilm angelöst wird, es gehen also Teile des Biofilms in das zirkulierte Gemisch über, ohne das die für den Biofilm charakteristischen dreidimensionalen Strukturen chemisch aufgelöst werden.

In Verfahrensschritt 6 wird eine Probe des zirkulierten Gemischs entnommen, die aufgrund der oben beschriebenen Enzymaktivität Teile des Biofilms enthält, sofern dieser in der Anlage vorhanden ist. In einem optionalen Verfahrensschritt 7 kann der Anteil organischer Rückstände bereits quantitativ anhand einer Probe bestimmt werden. Dazu kann beispielweise ein TOC- oder ein CSB-Verfahren angewandt und dessen Ergebnis mit einem vorgegebenen Grenzwert verglichen werden. Kann in diesem optionalen Verfahrensschritt bereits festgestellt werden, dass nicht genügend organische Stoffe zur Bildung eines Biofilms in dem zirkulierten Gemisch vorliegen, kann bereits das Nichtvorhandensein eines Biofilms festgestellt und das Verfahren an dieser Stelle in Verfahrensschritt 11 unter Ausgabe des Nicht-Vorhandenseins eines Biofilms beendet werden. Werden aber genug organische Stoffe zur Bildung eines Biofilms detektiert, kann mit dem optionalen Verfahrensschritt 8 oder gleich mit dem Verfahrensschritt 9 fortgefahren werden. Alternativ oder zusätzlich wird ein Teil der Probe im optionalen Schritt 7 einem PCR-.Verfahren unterzogen, bei dem ein Abschnitt der Desoxyribonukleinsäure eines Pathogens, welches im Biofilm enthalten ist, ausgewählt und durch das PCR-Verfahren repliziert wird. Anhand der am Ende des PCR-Verfahrens erhaltenen Quantität des Abschnitts kann das Vorhandensein von Pathogenen, die diesen Abschnitt aufweisen, bestimmt werden.

In dem optionalen Verfahrensschritt 8 kann die Probe zu einem Filterkuchen filtriert werden um den Anteil an den zu detektierenden Stoffen im Probenvolumen zu erhöhen.

Die Probe wird anschließend bei Verfahrensschritt 9 unter Verwendung üblicher Protokolle mit Fluoreszenzmarkern versehen. Bei der hier beschriebenen Ausführungsform werden direkt emittierende Fluoreszenzmarker verwendet, wobei die unterschiedlichen zu detektierenden Komponenten des Biofilms spezifisch mit Fluoreszenzmarkierungen charakteristischer Emissionswellenlängen markiert und anschließend über Fluoreszenzmikroskopie detektiert werden. Alternativ können beispielsweise indirekt emittierende Fluoreszenzmarker verwendet werden, die Detektionssubstrat in einer enzymatischen Reaktion umsetzen, sodass das Detektionssubstrat Licht emittiert. In Verfahrensschritt 10 wird ein Abbild der Probe erstellt und ausgewertet. Dazu können vorzugsweise unterschiedliche Fluoreszenzmarker verwendet werden, die aufgrund der unterschiedlichen Emissionswellenlängen leicht unterschieden werden können. Das Abbild kann quantitativ ausgewertet werden. Das bedeutet, dass für jeden Fluoreszenzmarker die photonische Ausbeute quantifiziert und dadurch das Vorhandensein der verschiedenen mit Fluoreszenzmarker gebundenen Bestandteile des Biofilms detektiert werden kann. Dazu kann beispielsweise die quantifizierte photonische Ausbeute für einen Fluoreszenzmarker mit einem vorgegebenen Referenzwert verglichen und ein Erkennungssignal dann ausgegeben werden, wenn die Ausbeute den Referenzwert übersteigt. Hier empfiehlt es sich, die an der Probe gemessenen Werte mit einem Referenzbild zu vergleichen, welches nach demselben Protokoll wie die Probe markiert und gewaschen wurde, aber keine Bindungspartner für die Fluoreszenzmarkierungen enthält. Außerdem kann aus diesen gemessenen Werten einfach und ohne Kalibration ein Verhältnis der verschiedenen Bestandteile des Biofilms abgeschätzt werden. Das Abbild kann weiters auch qualitativ untersucht werden, da die fluoreszenzmarkierten Zellen und die fluoreszenzmarkierte dreidimensionale Struktur auf dem Abbild erkennbar sind. Auch hier empfiehlt es sich, das Abbild mit dem Vergleichsabbild eines Biofilms zu vergleichen, was entweder durch den Benutzer oder durch einen automatisierten Vergleichsalgorithmus erfolgen kann. Alternativ zur Fluoreszenzmikroskopie kann beispielsweise ein enzymgebundenes Immunosorbentnachweisverfahren (ELISA) durchgeführt werden, wobei hier bevorzugt indirekt emittierende Fluoreszenzmarker verwendet werden. Wird zufolge der Auswertung festgestellt, dass kein Biofilm vorliegt, wird dies in Verfahrensschritt 11 ausgegeben. Wird ein Biofilm detektiert, wird in Verfahrensschritt 12 ein Erkennungssignal ausgegeben.

## Patentansprüche

1. Verfahren zum Erkennen eines Biofilms in geschlossenen Anlagen, bei dem zuerst ein Nachspülmedium in die entleerte Anlage eingebracht (1) und anschließend eine enzymatische Lösung zum Anlösen des Biofilms beigemischt wird (3) und das Gemisch in der Anlage zirkuliert (4), **dadurch gekennzeichnet, dass** der Anlage eine Probe des zirkulierten Gemischs entnommen wird (6), welche mit Fluoreszenzmarkern fluoreszenzmarkiert (9) wird, die eine Biofilmkomponente spezifisch binden und ein photometrisches Abbild der Probe erstellt wird (10), wonach ein Erkennungssignal dann ausgegeben wird, wenn im Abbild die charakteristischen Emissionswellenlängen jener Fluoreszenzmarker detektiert werden, mit der die in der Probe vorhandenen fluoreszenzmarkierten Biofilmkomponenten markiert sind.

2. Verfahren nach Anspruch 1, **dadurch gekennzeichnet, dass** das Abbild der Probe mittels Fluoreszenzmikroskopie erstellt wird.

3. Verfahren nach Anspruch 1 oder 2, **dadurch gekennzeichnet, dass** das Nachspülmedium vor dem Beimischen der enzymatischen Lösung auf 40-50°C temperiert wird (2).

4. Verfahren nach einem der Ansprüche 1 bis 3, **dadurch gekennzeichnet, dass** der Anteil organischer Rückstände in der Probe vor dem Erstellen des Abbildes quantitativ bestimmt wird (7) und kein Biofilm detektiert wird, wenn der Anteil einen vorgegebenen Grenzwert unterschreitet.

5. Verfahren nach einem der Ansprüche 1 bis 4, **dadurch gekennzeichnet, dass** die enzymatische Lösung Amylasen und/oder Proteasen und/oder Saccharasen umfasst.

6. Verfahren nach einem der Ansprüche 1 bis 5, **dadurch gekennzeichnet, dass** das Gemisch vor der Probenentnahme bei konstanter Temperatur wenigstens eine Stunde in der geschlossenen Anlage zirkuliert.

7. Verfahren nach einem der Ansprüche 1 bis 6, **dadurch gekennzeichnet, dass** der pH-Wert des Gemisches verändert wird und dass Gemisch mit verändertem pH-Wert wenigstens eine Stunde in der geschlossenen Anlage zirkuliert (5), bevor die Probe entnommen wird.

8. Verfahren nach einem der Ansprüche 1 bis7, **dadurch gekennzeichnet, dass** die Probe ein durch Filtration zumindest eines Teils des zirkulierten Gemischs gewonnener Filterkuchen ist.

9. Verfahren nach einem der Ansprüche 1 bis 8, **dadurch gekennzeichnet, dass** ein Teil der Probe einem Polymerasekettenreaktionsprozess unterzogen wird, bei dem ein bestimmter Desoxyrobonukleinsäurestrangabschnitt als Biofilmkomponente vervielfältigt wird.

10. Verfahren nach einem der Ansprüche 1 bis 9, **dadurch gekennzeichnet, dass** das Abbild der Probe als Teil eines enzymgebundenen Immunosorbentnachweisverfahrens erstellt wird.

11. Verfahren nach Anspruch 10, **dadurch gekennzeichnet, dass** das enzymgebundene Immunosorbentnachweisverfahrens ein Sandwichenzymgebundenes Immunosorbentnachweisverfahren ist.

## Claims

1. Method for detecting a biofilm in closed plants, in which first a rinsing medium is introduced into the emptied plant (1) and then an enzymatic solution for dissolving the biofilm is added (3) and the mixture is circulated in the plant (4), **characterized in that** a sample of the circulated mixture is taken from the plant (6), which sample is fluorescently labelled (9) with fluorescent markers which specifically bind to a biofilm component, and a photometric image of the sample is produced (10), after which a recognition signal is output when the characteristic emission wavelengths of those fluorescent markers with which the fluorescently labelled biofilm components present in the sample are labelled are detected in the image.

2. Method according to claim 1, **characterized in that** the image of the sample is produced by means of fluorescence microscopy.

3. Method according to claim 1 or 2, **characterized in that** the rinsing medium is tempered to 40-50°C before the enzymatic solution is added (2).

4. Method according to one of claims 1 to 3, **characterized in that** the proportion of organic residues in the sample is quantitatively determined (7) before the image is created and no biofilm is detected if the proportion falls below a predetermined limit value.

5. Method according to one of claims 1 to 4, **characterized in that** the enzymatic solution comprises amylases and/or proteases and/or saccharases.

6. Method according to one of claims 1 to 5, **characterized in that** the mixture circulates in the closed plant at a constant temperature for at least one hour before sampling.

7. Method according to one of claims 1 to 6, **characterized in that** the pH value of the mixture is changed and that the mixture with the changed pH value is circulated in the closed plant for at least one hour (5) before the sample is taken.

8. Method according to one of claims 1 to 7, **characterized in that** the sample is a filter cake obtained by filtration of at least part of the circulated mixture.

9. Method according to any one of claims 1 to 8, **characterized in that** part of the sample is subjected to a polymerase chain reaction process in which a specific deoxyribonucleic acid strand section is amplified as a biofilm component.

10. Method according to any one of claims 1 to 9, **characterized in that** the image of the sample is produced as part of an enzyme-linked immunosorbent detection process.

11. Method according to claim 10, **characterized in that** the enzyme-linked immunosorbent detection method is a sandwich enzyme-linked immunosorbent detection method.

## Revendications

1. Procédé de détection d'un biofilm dans des installations fermées, dans lequel un milieu de rinçage est d'abord introduit à l'intérieur de l'installation vidée (1) et une solution enzymatique est ensuite incorporée dans le but de dissoudre un biofilm (3), et enfin le mélange est mis en circulation à l'intérieur de l'installation (4), **caractérisé en ce qu'**un échantillon du mélange mis en circulation (6) est prélevé depuis l'installation et est marqué par fluorescence (9) avec des marqueurs fluorescents qui lient spécifiquement un composant du biofilm, et qu'une image photométrique de l'échantillon est générée (10), après quoi un signal de détection est émis lorsque les longueurs d'onde d'émission caractéristiques des marqueurs fluorescents utilisés pour marquer les composants du biofilm marqués par fluorescence présents dans l'échantillon sont détectées à l'intérieur de l'image.

2. Procédé selon la revendication 1, **caractérisé en ce que** l'image de l'échantillon est générée au moyen d'une microscopie à fluorescence.

3. Procédé selon la revendication 1 ou 2, **caractérisé en ce que** le milieu de rinçage est tempéré (2) à 40-50°C avant que la solution enzymatique soit ajoutée.

4. Procédé selon l'une quelconque des revendications 1 à 3, **caractérisé en ce que** la part de résidus organiques dans l'échantillon est déterminé de façon quantitative (7) avant que l'image soit générée, et **en ce qu'**aucun biofilm n'est détecté si la part est inférieure à une valeur limite prédéterminée.

5. Procédé selon l'une quelconque des revendications 1 à 4, **caractérisé en ce que** la solution enzymatique comprend des amylases et/ou des protéases et/ou des saccharases.

6. Procédé selon l'une quelconque des revendications 1 à 5, **caractérisé en ce que** le mélange circule à l'intérieur de l'installation fermée à une température constante pendant au moins une heure avant que l'échantillon soit prélevé.

7. Procédé selon l'une quelconque des revendications 1 à 6, **caractérisé en ce que** le pH du mélange est modifié, et **en ce que** le mélange à pH modifié circule (5) à l'intérieur de l'installation fermée pendant au moins une heure avant que l'échantillon soit prélevé.

8. Procédé selon l'une quelconque des revendications 1 à 7, **caractérisé en ce que** l'échantillon est un gâteau de filtration qui est obtenu en filtrant au moins une partie du mélange mis en circulation.

9. Procédé selon l'une quelconque des revendications 1 à 8, **caractérisé en ce qu'**une partie de l'échantillon est soumise à un procédé de réaction en chaîne par polymérase dans lequel une section de brin d'acide désoxyrobonucléique particulier est amplifiée en tant que composant du biofilm.

10. Procédé selon l'une quelconque des revendications 1 à 9, **caractérisé en ce que** l'image de l'échantillon est générée comme partie d'un procédé de détection d'immunosorbant lié à un enzyme.

11. Procédé selon la revendication 10, **caractérisé en ce que** le procédé de détection d'immunosorbant lié à un enzyme est un procédé de détection d'immunosorbant lié à un enzyme en sandwich.
